# EUROPEAN PATENT APPLICATION

(11) **EP 2 777 742 A2**
(43) Date of publication of application: **17.09.2014**
(21) Application number: 14158447.4
(22) Date of filing: 07.03.2014
(51) Int. Cl.: A61M 25/00

(54) **Catheter system**

(30) Priority: 12.03.2013 US 201361777236 P
(71) Applicant: St. Jude Medical, Cardiology Division, Inc., St. Paul MN 55117-9913 (US)
(72) Inventor: Goodman, James Paul, Eden Prairie, MN Minnesota 55344 (US); Park, Kevin Gin, Golden Valley, MN Minnesota 55422 (US); Knippel, Bradley C., Lino Lakes, MN Minnesota 55014 (US)
(74) Representative: Smaggasgale, Gillian Helen

(57) **Abstract**

In a catheter system and method for controlling such a system, a first working component is carried by a shaft and has at least one characteristic that is adjustable. A first control line extends within the shaft and operatively couples a first actuator with the first working component such that actuation of the first actuator acts on the first control line to adjust the first working component. A second control line separate from the first control line extends within the shaft and is operatively coupled to a second working component such that acting on the second control line adjusts at least one characteristic of the second working component. A compensator assembly is associated with the handle, with the second control line being operatively coupled to the compensator assembly. The compensator assembly is operable, in response to actuation of the first actuator, to inhibit slack from forming in the second control line.

## Description

### BACKGROUND OF THE DISCLOSURE

### A. Field of the disclosure

The present disclosure relates generally to a catheter system for use in a human body, and more particularly to a catheter system having at least one selectively adjustable feature, and still more particularly to a catheter system having multiple control lines associated with multiple components of the system, at least one of which is selectively adjustable.

### B. Background Art

Catheter systems are well known in the art for use in medical procedures, such as diagnostic, therapeutic and ablative procedures. Typical catheter systems generally include an elongate catheter extending from a handle. A physician manipulates the catheter through the patient's vasculature to an intended site within the patient. The catheter typically carries one or more working components, such as electrodes or other diagnostic, therapeutic or ablative devices for carrying out the procedures. Controls, or actuators may be provided on the handle for selectively adjusting one or more characteristics of the working components.

One particular example of the catheter system is an ablative catheter system in which the working component is a multi-electrode component carried at the distal end of the catheter. A control wire extends within the shaft of the catheter from the electrode component to the handle to operatively connect the electrode component to an actuator on the handle. Actuation of the actuator acts on the control wire to configure the electrode component into a desired configuration. For example, in one such ablative catheter system made by St. Jude Medical, Inc. under the trade name EnligHTN, the multi-electrode component is in the form of an electrode basket. Upon locating the electrode basket at a desired location within the patient, actuation of the actuator on the handle pulls on the control wire to reconfigure the electrode from a collapsed configuration to an expanded configuration in which the electrodes are in contact with a surface, such as an arterial wall. It is thus important to maintain proper tension in the control wire. In some catheter systems, there may be a need for two or more separate control wires, such as where there are two or more working components carried by the catheter. In such an arrangement, it is desirable that proper tension in each of the control wires be maintained, particularly when only one of the control wires is being acted upon. It is also desirable to maintain a secure connection of the catheter to the handle. It is further desirable for the physician to be able to readily actuate the actuator, and for the system to facilitate maintaining the actuator in a desired position corresponding to a desired configuration of a working component.

### BRIEF SUMMARY OF THE DISCLOSURE

In one embodiment, a catheter system generally comprises a handle and an elongate hollow shaft having a proximal end connected to the handle and a distal end remote from the handle. A first working component is carried by the shaft and has at least one characteristic that is adjustable. A first actuator is associated with the handle for selectively adjusting at least one characteristic of the first working component. A first control line extends at least in part within the shaft and operatively couples the first actuator with the first working component such that actuation of the first actuator acts on the control line to adjust the at least one characteristic of the first working component. A second working component carried by the shaft has at least one characteristic that is adjustable. A second control line separate from the first control line extends at least in part within the shaft. The second control line is operatively coupled to the second working component such that acting on the second control line adjusts at least one characteristic of the second working component. A compensator assembly is associated with the handle, with the second control line being operatively coupled to the compensator assembly. The compensator assembly is operable, in response to actuation of the first actuator, to inhibit slack from forming in the second control line.

A first actuator is associated with the handle for selectively adjusting at least one characteristic of the shaft. A first control line extends at least in part within the shaft, with the first control line operatively coupling the first actuator with the shaft such that actuation of the first actuator acts on the control line to adjust the at least one characteristic of the shaft. A working component is disposed either at the distal end of the shaft or intermediate the distal end and proximal end of the shaft. A second control line separate from the first control line extends at least in part within the shaft. The second control line is operatively coupled to the working component such that acting on the second control line adjusts at least one characteristic of the working component. A compensator assembly is associated with the handle, with the second control line being operatively coupled to the compensator assembly. The compensator assembly is responsive to actuation of the first actuator to inhibit slack from forming in the second control line.

In one embodiment of a method of controlling a catheter system of the type having a handle, a first component operatively coupled to the handle by first control line, and a second component operatively coupled to the handle by a second control line, the handle is operated to act on the first control line whereby acting on the first control line adjusts at least one characteristic of the first component. The second control line is automatically acted on, in response to operating the handle to act on the first control line, to inhibit slack from forming in the second control line upon adjustment of the at least one characteristic of the first component.

In another embodiment, an electrode catheter system generally comprises a handle having a housing and a longitudinal axis. An elongate, hollow flexible shaft has a proximal end connected to the handle and a distal end remote from the handle. A deflectable segment of the shaft is deflectable relative to a remaining segment of the shaft. A shaft actuator is associated with the handle for selectively deflecting the deflectable segment of the catheter shaft. The shaft actuator at least in part comprises a first shuttle disposed within and moveable longitudinally relative to the handle housing. A shaft pull wire extends within the catheter shaft and is connected to the deflectable segment of the catheter shaft. The shaft pull wire is operatively coupled to the first shuttle such that actuation of the shaft actuator drives longitudinal movement of the first shuttle relative to the handle housing so as to pull on the shaft pull wire to deflect the deflectable segment of the catheter shaft. An electrode component is carried by the catheter shaft and is configurable from a collapsed configuration to an expanded configuration. An electrode pull wire separate from the shaft pull wire extends at least in part within the shaft and is operatively coupled to the electrode component. A second shuttle is disposed within and moveable longitudinally relative to the handle housing and is also moveable longitudinally relative to the first shuttle. The electrode pull wire is operatively coupled to the second shuttle. The second shuttle is responsive to longitudinal movement of the first shuttle to move longitudinally relative to the first shuttle so as to inhibit slack from forming in the electrode pull wire.

The foregoing and other aspects, features, details, utilities and advantages of the present disclosure will be apparent from reading the following description and claims, and from reviewing the accompanying drawings.

### BRIEF DESCRIPTION OF THE DRAWINGS

Figure 1 is a perspective view of one embodiment of a catheter system;
Figure 2 is a side elevation of a catheter and handle of the catheter system of Figure 1, with a distal or front end segment of a catheter shaft deflected relative to the remainder of the catheter shaft and with a slide actuator in its extended or actuated position corresponding to the deflection of the catheter shaft;
Figure 3 is a side elevation similar to Figure 2, but with the slide actuator in its neutral or unextend position corresponding to the catheter shaft being undeflected, and with an electrode basket of the catheter system in an expanded configuration resulting from rotation of a rotatable actuator;
Figure 4 is a cross-section of a portion of the handle of the catheter system of Figure 1;
Figure 5 is a cross-section of another portion of the handle of the catheter system of Figure 1;
Figure 6 is an exploded view of the handle of the catheter system of Figure 1;
Figure 7 is an enlarged portion of the cross-section of Figure 4;
Figure 8 is a cross-section of the handle taken perpendicular to the cross-section of Figure 7;
Figure 9 is a top plan view of an assembled worm gear housing, worm gear assembly and pinion member;
Figure 9A is a top plan view of the worm gear housing,
Figure 10 is a bottom plan view of the assembled worm gear housing, worm gear assembly and pinion member;
Figure 10A is a bottom plan view of the worm gear housing;
Figure 11 is a top plan view of a bottom half of the worm gear housing;
Figure 12 is an exploded perspective view of a worm gear assembly of the handle of the catheter system of Figure 1;
Figure 13 is a top plan view of a pinion member of the handle;
Figure 14 is a front elevation of the pinion member of Figure 13;
Figure 15 is a bottom plan view of a top half of a barrel housing of the handle;
Figure 16 is a top plan view of a bottom half of the barrel housing of the handle;
Figure 17 is a top plan view of the bottom half of the barrel housing with the bottom half of the worm gear housing, the worm gear assembly and the pinion member disposed therein;
Figure 18 is a top plan view of a bottom half of the handle housing, with a bottom half of the barrel housing and the entire worm gear housing and related internal components disposed therein;
Figure 19 is a top plan view of the bottom half of the handle housing, with the entire barrel housing, worm gear housing and related internal components disposed therein;
Figure 20 is a top plan view of the bottom half of the handle housing, with the bottom half of the barrel housing, the bottom half of the worm gear housing, the worm gear assembly and the pinion member disposed therein, the pinion member being in an initial or neutral position corresponding to an undeflected configuration (Figure 1) of the catheter shaft;
Figure 21 is a top plan view similar to Figure 20 with the pinion member pivoted relative to the handle housing to a maximum pivoted position corresponding to the maximum deflected configuration (Figure 2) of the catheter shaft;
Figure 21A is an enlarged cross-section of a portion of the handle of Figure 4;
Figure 22 is a perspective view of a flex relief member of the handle of the catheter system of Figure 1;
Figure 23 is a rear elevation thereof;
Figure 24 is a perspective view of a shaft collar of the handle of the catheter system of Figure 1;
Figure 25 is a cross-section thereof;
Figure 26 is a cross-section thereof taken normal to the cross-section of Figure 22; and
Figure 27 is a perspective cross-section of the flex relief member and shaft collar with the catheter shaft connected thereto.

Corresponding reference characters indicate corresponding parts throughout the several views of the drawings.

### DETAILED DESCRIPTION OF THE DISCLOSURE

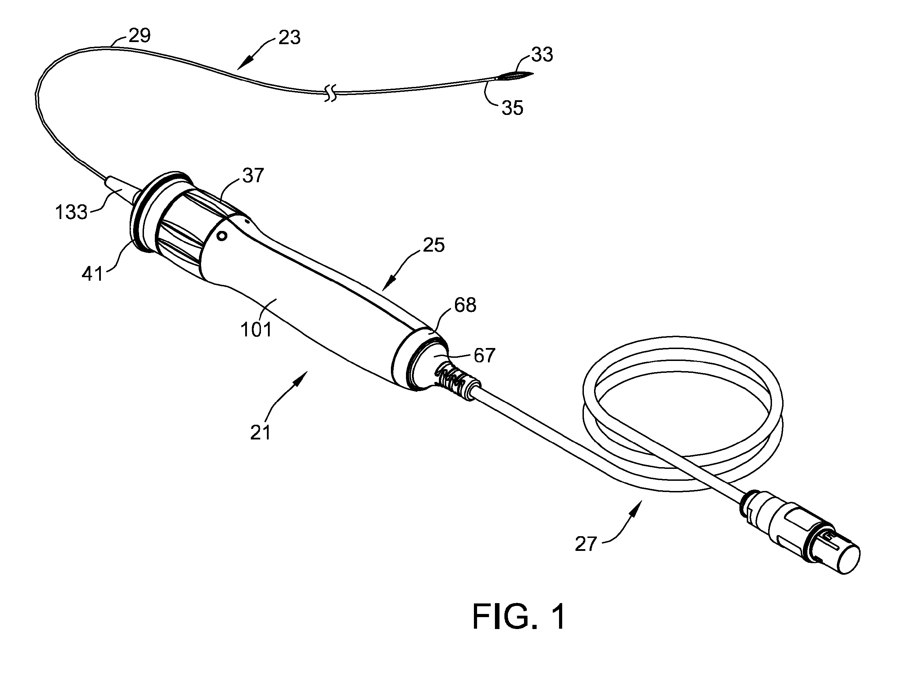

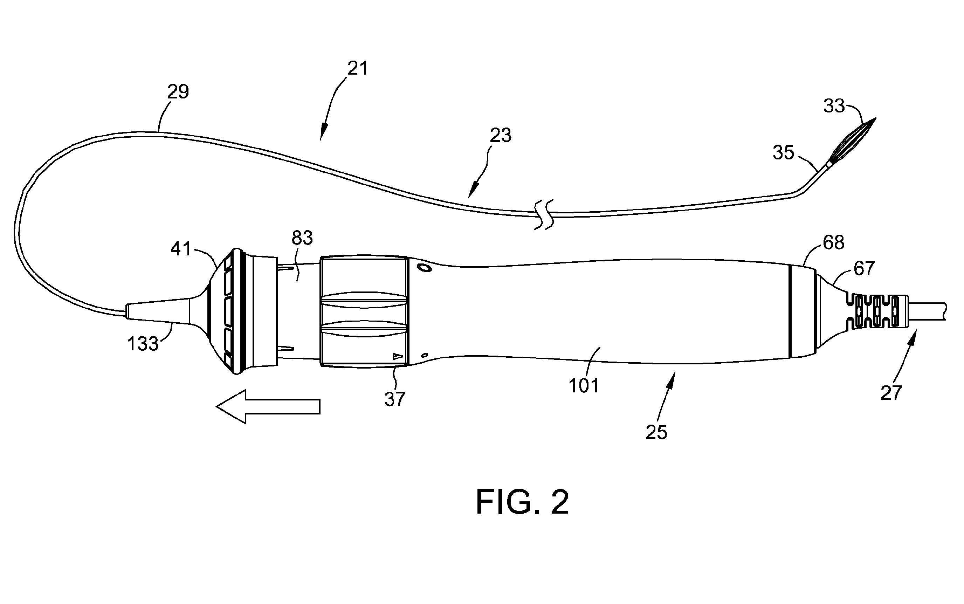

Referring now to the drawings, and in particular to Figures 1 and 2, one embodiment of a catheter system is indicated generally at 21 and includes a catheter 23, a handle 25 to which the catheter is connected, and a conductor assembly 27 for electrically connecting the catheter system to a suitable power supply (not shown).

In the embodiments illustrated and described herein, the catheter system 21 includes an elongate flexible catheter 23 that is also selectively deflectable (e.g., bendable) - such as at or adjacent the end or tip, broadly referred to as a first working component or first component, of the catheter - as illustrated for example in Figure 2. The catheter system 21 also includes what is broadly referred to as a second working component (or second component). As used herein, a working component is intended to refer to any component that is used for guiding, diagnostic, therapeutic, ablative or other function relating to a patient. Working components may be carried by the catheter 23 and selectively operated or adjusted. As used in herein, selective operation or adjustment of a working component is intended to refer to a functional changing of at least one characteristic of the working component, such as changing the configuration of the component, changing the orientation of the component, supplying current to the component, inflating or collapsing the component or otherwise adjusting, manipulating or operating the component for its intended purpose.

As one example, the catheter system 21 illustrated and described herein is suitably constructed for use as an ablation system, such as a renal or heart ablation system. More particularly, the illustrated catheter system 21 is a multi-electrode renal denervation system. One example of such a system is that currently made by St. Jude Medical, Inc. under the trade name EnligHTN. General operation of a multi-electrode renal denervation system is known to those of skill in the art and is not described further herein except to the extent necessary to describe the present embodiments. It is understood that the catheter system 21 may be used for any other suitable treatment or purpose without departing from the scope of this disclosure. Additionally, while the catheter system 21 is illustrated and described herein as including only the flexible catheter, the system may further include other components used, for example, to guide the flexible catheter into the patient - such as, without limitation, a relatively more rigid guide catheter (not shown).

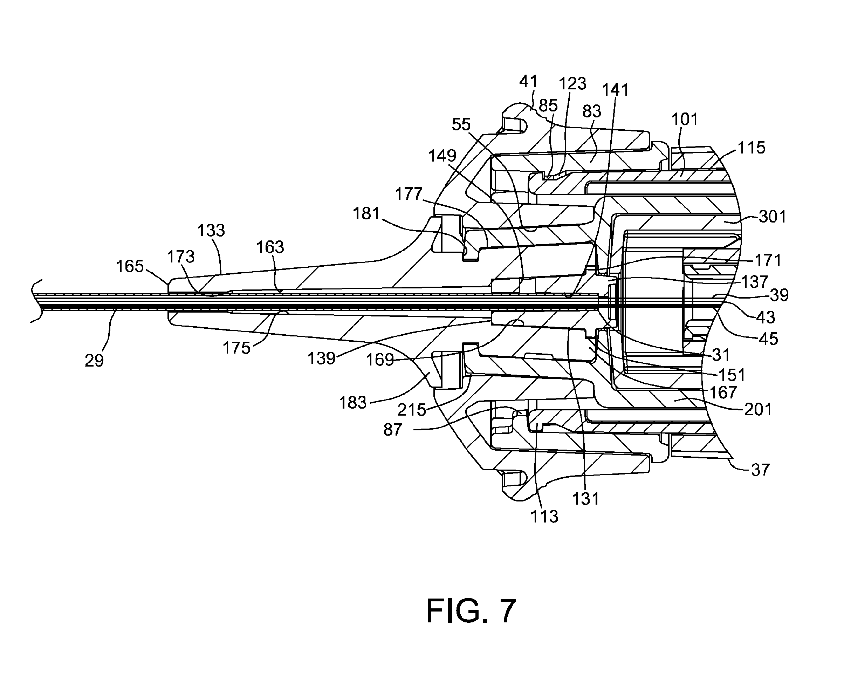

The illustrated catheter 23 of Figure 1 includes an elongate, flexible hollow shaft 29 having a central passage and connected to the handle 25 at or near a proximal or rear end 31(not visible in Figures 1 and 2 but seen, e.g., in Figure 7) of the catheter shaft, and an electrode basket 33 (broadly, a working component and more broadly a second component of the catheter system) disposed at or near a distal or front end 35 (or what is sometimes referred to as the tip) of the catheter shaft. It is understood, however, that the electrode basket 33 may be disposed anywhere along the catheter shaft 29 intermediate the rear end 31 and the front end 35 thereof without departing from the scope of this disclosure.

As used herein, the terms proximal and front, and distal and rear, are used with reference to the orientation of the catheter system 21 illustrated in the various drawings and for the purpose of describing the various embodiments set forth herein, and are not intended as limiting the catheter system and related components to having any particular orientation upon assembly or during operation thereof.

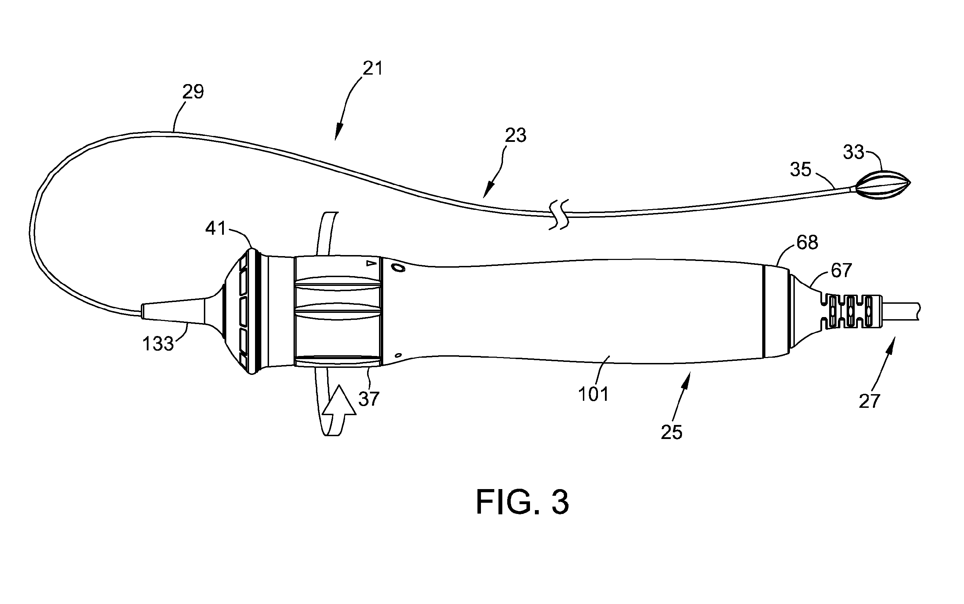

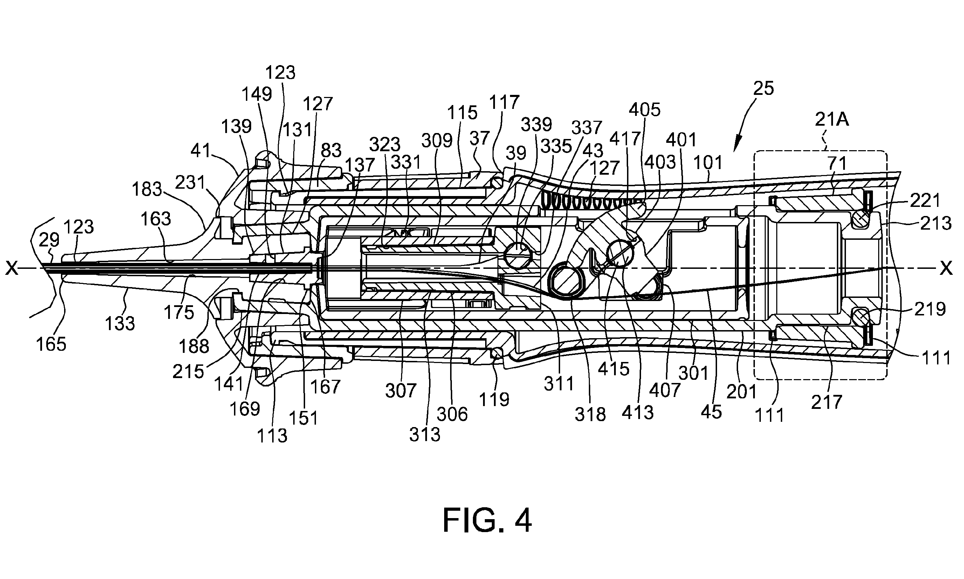

The electrode basket 33 is suitably configurable between a collapsed configuration (Figure 1) and an expanded configuration (Figure 3). An annular (e.g., ringshaped) actuator 37 (Figure 3) is mounted on the handle 25 for rotation relative thereto and is operatively connected to the electrode basket 33 for selectively configuring the electrode basket between its collapsed and expanded configurations. It is understood that other suitable actuators (e.g., slide, push button, lever, etc.) may be used instead of the rotating actuator 37 to selectively configure the electrode basket 33 without departing from the scope of this disclosure. In some embodiments, the electrode basket 33 may be selectively adjustable between an infinite number of configurations between its collapsed and expanded configurations using the actuator 37. A control line, such as a suitable cable or pull wire 39 (Figure 4), extends from the electrode basket 33 within the hollow catheter shaft 29 and into the handle 25 and operatively connects the annular actuator 37 with the electrode basket via a worm gear assembly 306 (Figure 4 and described in further detail later herein) to which the pull wire is connected. While in the illustrated embodiment a single pull wire 39 is used to selectively configure the electrode basket, it is contemplated that two or more pull wires, cables or other suitable control lines may be used for selectively configuring the electrode basket. It is also understood that the control line may be any suitable control line other than a pull wire, such as a cable, string, tie, compression member or other suitable line useful to operatively connect the electrode basket 33 to the worm gear assembly 36 and hence the handle 25.

In the illustrated embodiment, the catheter shaft 29 is also configured for deflection near the tip or front end 35 thereof, such as between an undeflected configuration (Figure 1) and a deflected (e.g., bent or angled) configuration (Figure 2) for use in guiding the catheter 23 into desired positions within the patient. As best seen in Figures 1 and 2, a suitable slide actuator 41 is mounted on the handle 25 for sliding movement longitudinally of the handle and is operatively connected to the deflectable segment of the catheter shaft 29 for movement between a first or neutral position (Figure 1) corresponding to the undeflected configuration of the catheter shaft and a second (e.g., extended) position (Figure 2) corresponding to the deflected configuration of the catheter shaft. The slide actuator 41 permits the catheter shaft 29 to be selectively deflected to any number of angular positions between the undeflected configuration and a predetermined maximum deflection (e.g., angular position) of the catheter. It is understood that any other suitable actuator (e.g., rotating, push button, lever, etc.) may be used to selectively adjust (e.g., deflect) the catheter shaft 29 without departing from the scope of this disclosure.

Another control line, such as a suitable cable or pull wire 43 (Figure 4), extends from the segment of the catheter shaft 29 that is deflectable (e.g., the front end 35 of the illustrated catheter shaft) within the hollow catheter shaft and into the handle 25 and operatively connects the slide actuator 41 with the deflectable segment of the catheter via a pinion member 401 (Figure 4 and described in further detail later herein) to which the pull wire is connected. While in the illustrated embodiment a single pull wire 43 is used to selectively deflect the catheter shaft 29, it is contemplated that two or more wires, cables or other suitable control lines may be used for selectively bending the catheter. It is also understood that the control line 43 may be any suitable control line other than a pull wire, such as a cable, string, tie, compression member or other suitable line useful to operatively connect the deflectable segment of the catheter shaft 29 to the pinion member 401 and hence the handle 25. The control line 43 associated with deflection of the catheter shaft 29 is different from the control line 39 associated with selectively configuring the electrode basket 33 to permit configuration of the electrode basket at least in part independent of the deflection of the catheter.

A conductive wire, or more particularly in the illustrated embodiment a twisted bundle 45 of two or more conductive wires (Figure 4) corresponding to the multiple electrodes of the electrode basket 33, extends from the electrode basket within the catheter shaft 29 and into the handle 25 for electrical connection with the conductor assembly 27 to provide electrical communication between the power supply and the electrode basket. It is understood that the power supply may be any power supply, such as ultrasonic, RF or other suitable power supply.

With particular reference now to Figures 4-6, the handle 25 has a longitudinal or lengthwise axis X and generally comprises an outermost housing, referred to herein as a handle housing 101, an intermediate housing, referred to herein as a barrel housing 201 extending longitudinally within the handle housing and being slidable longitudinally relative to the handle housing to broadly define an outer shuttle, and an innermost housing, referred to herein as a worm gear housing 301 extending longitudinally within the barrel housing and being slidable longitudinally relative to both the barrel housing and the handle housing to broadly define an inner shuttle. The illustrated handle housing 101 is of two piece construction (e.g., what is referred to herein as a top half 103 and a bottom half 105 of the handle housing). However, the handle housing 101 may be of any suitable alternative construction, such as of a single-piece construction or of more than two pieces.

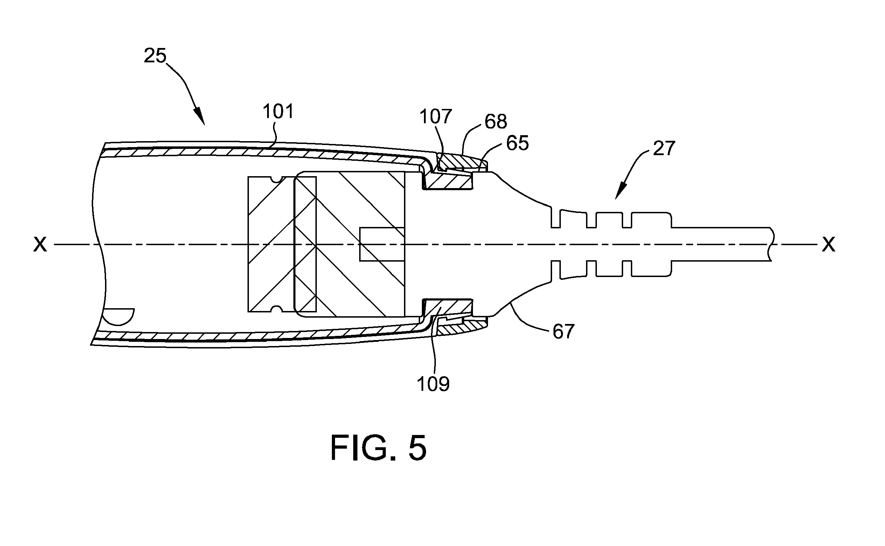

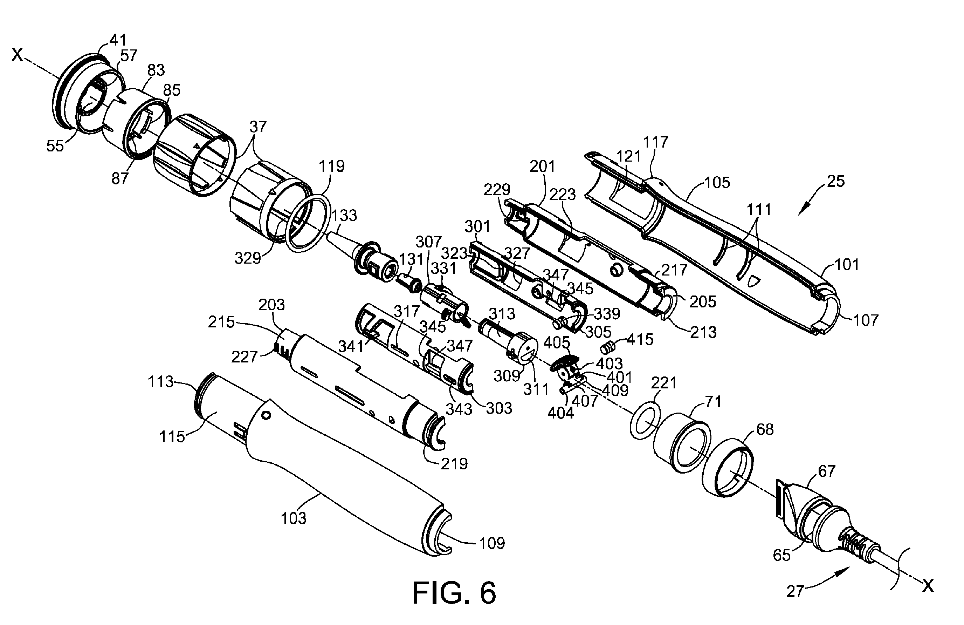

The handle housing 101 has a distal or rear end 107 (Figure 5) to which the conductor assembly 27 is connected in any suitable manner. In the illustrated embodiment of Figure 5, for example, connection is by an inward tapered collar 109 at the rear end 107 of the handle housing 101 seating within an annular channel 65 formed in a connection plug 67 of the conductor assembly 27. A retaining ring 68 seats over the tapered collar 109 to generally close the rear end 107 of the handle housing 101. As seen in Figures 4 and 6, the handle housing 101 includes a pair of internal arcuate ribs 111 extending radially inward from the inner surface of the handle housing. These ribs 111 extend circumferentially about the inner surface of the handle housing 101 to longitudinally locate and retain a slide bearing 71 within the handle housing.

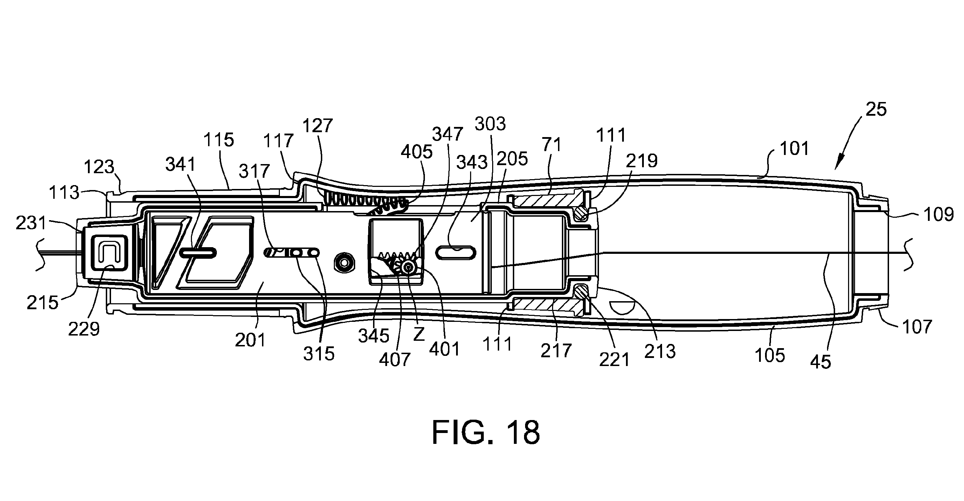

The handle housing 101 is configured adjacent its front end 113 as a cylindrical mount 115 for rotatably mounting the annular actuator 37 on the handle housing. A shoulder 117 (Figures 4 and 18) is formed in the outer surface of the mount 115 to function as a stop to facilitate longitudinal positioning of the annular actuator 37 on the handle housing 101. The shoulder 117 also accommodates a suitable sealing ring 119 (e.g., an elastomeric gasket or O-ring; Figures 4 and 6) to seal the interface between the annular actuator 37 and the handle housing 101. An opening 121 (Figure 6) is formed in the bottom half 105 of the handle housing 101 at the cylindrical mount 115 to facilitate operative connection of the annular actuator 37 with the electrode basket 33 via the worm gear assembly 306 as described in detail later herein.

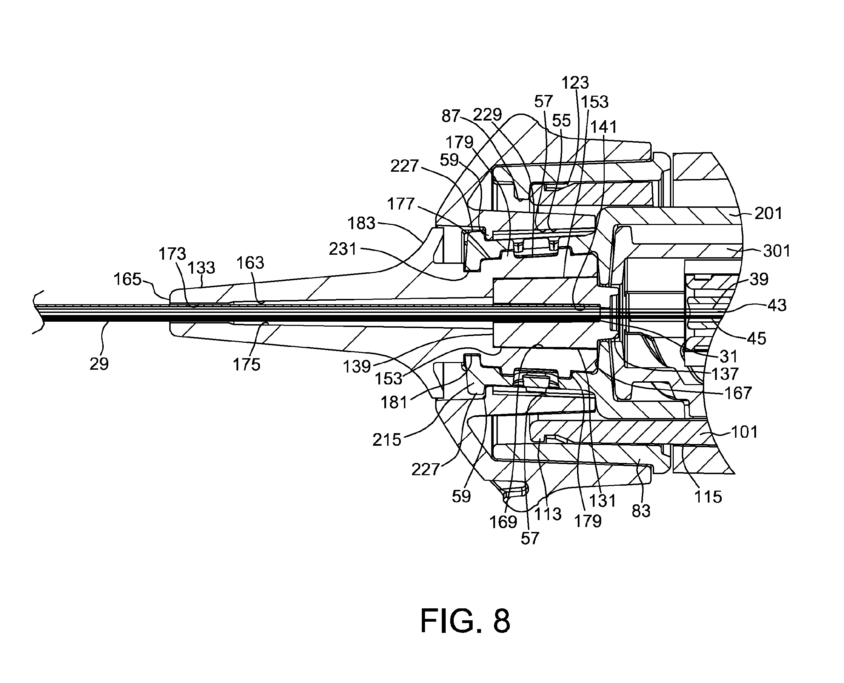

An annular groove 123 is formed in the outer surface of the handle housing 101 to facilitate mounting a sleeve 83 on the front end 113 of the handle housing. The sleeve 83, as illustrated in Figures 4 and 6-8, has a first set of internal projections 85 (Figures 6 and 7) extending radially inward from the inner surface of the sleeve. These projections 85 seat within the annular groove 123 to mount the sleeve 83 on the handle housing 101. A second set of internal projections 87 (one of which is illustrated in Figure 7 and the other in Figure 8) extends radially inward from the inner surface of the sleeve 83 in longitudinally spaced relationship with the first set of internal projections 85, and more particularly nearer to a front end of the sleeve. With the sleeve 83 mounted on the handle housing 101, the second set of internal projections 87 abuts against the front end 113 of the handle housing as illustrated in Figures 4, 7 and 8 to secure the sleeve on the handle housing against longitudinal movement relative thereto.

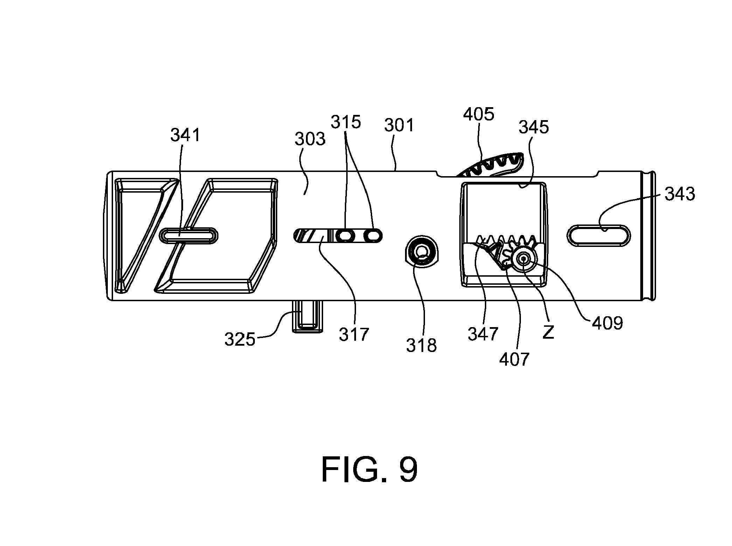

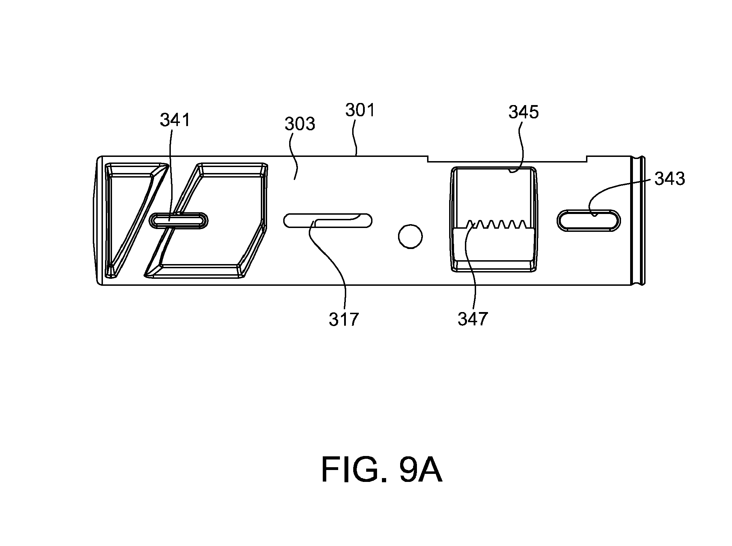

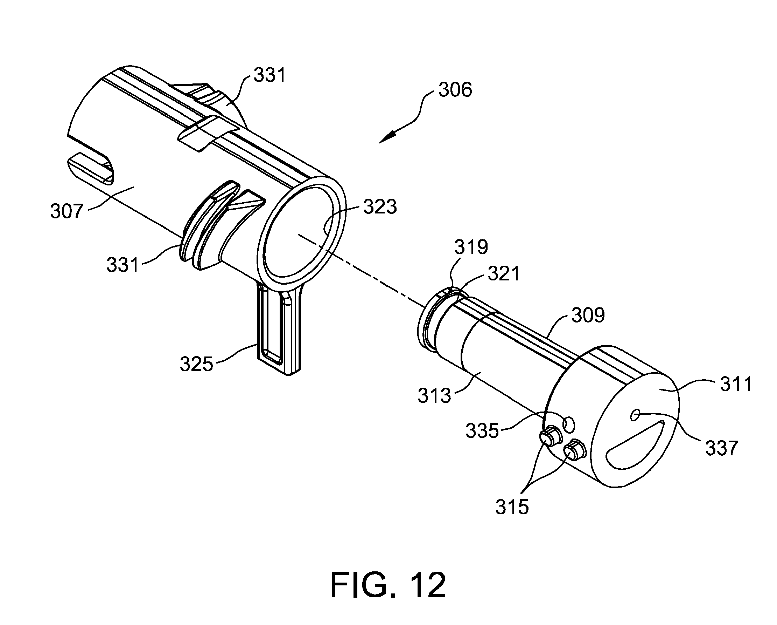

Referring now to Figures 9-12, the illustrated worm gear housing 301 (broadly, the inner shuttle of the handle 25) is of two-piece construction, referenced herein as a top half 303 and a bottom half 305. In other suitable embodiments the worm gear housing 301 may be of single-piece construction, or constructed of more than two pieces. The worm gear assembly 306 (Figures 9 and 12) is positionable longitudinally within the worm gear housing 301 and includes a worm gear 307 rotatable on a linear bushing 309. The linear bushing 309 has a head 311, and a smaller diameter shaft 313 extending longitudinally forward from the head. A pair of locating pins 315 project from the outer circumference of the head 311 for seating in a longitudinally extending slot 317 (as illustrated best in Figure 9) in the top half 303 of the worm gear housing 301 to locate and retain the worm gear assembly 306 in the worm gear housing and to inhibit the head 311 of the worm gear bushing 309 against rotation relative to the worm gear housing. The slot 317 is sized in length to permit longitudinal translation of the worm gear assembly 306 relative to the worm gear housing 301 in response to rotation of the worm gear 307 on the bushing 309. A distal or front end 319 of the bushing shaft 313 has an annular groove 321 formed therein to facilitate mounting of the worm gear 307 on the bushing 309.

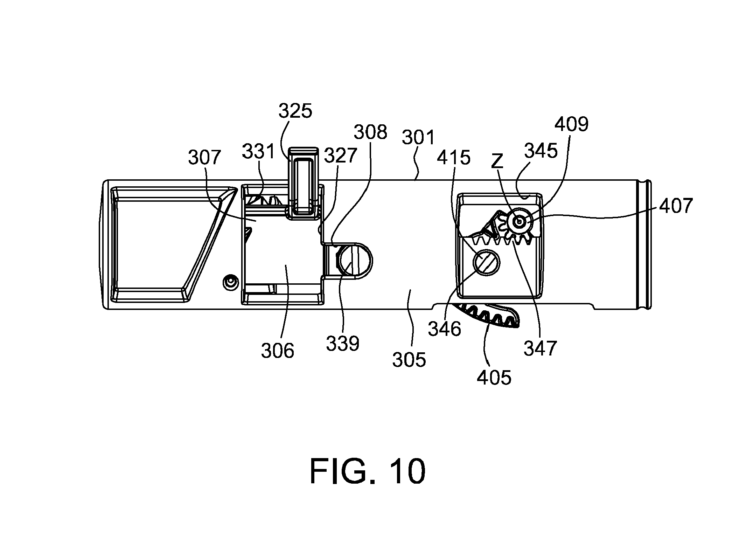

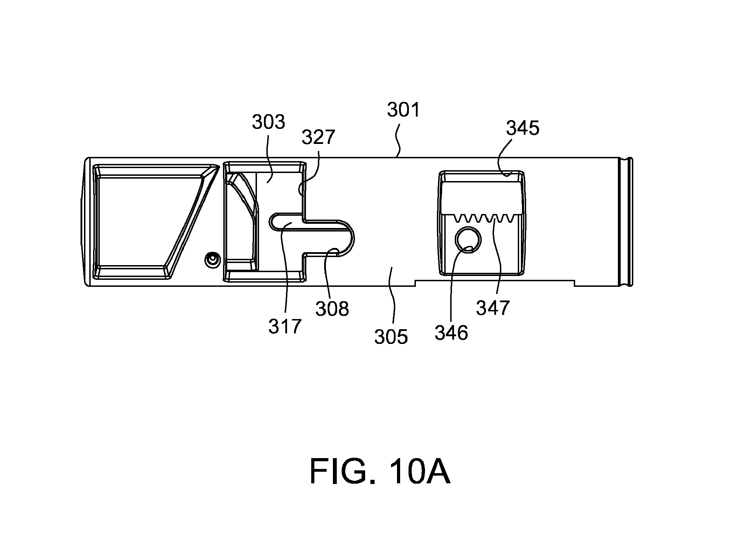

The illustrated worm gear 307 is generally tubular, having a central channel 323 for receiving the bushing shaft 313 therein. Catches (not shown) project radially inward of the channel 323 from the inner surface of the worm gear 307 for seating in the annular groove 321 of the bushing shaft 313 to mount the worm gear on the bushing for rotation on the shaft of the bushing. A lever arm 325 extends radially outward from the worm gear 307 for operative connection with the annular actuator 37. In particular, the bottom half 305 of the worm gear housing 301 has a window 327 (Figures 6, 10 and 11) through which the worm gear lever arm 325 extends when the worm gear assembly 306 is otherwise housed within the worm gear housing as illustrated in Figure 10. A longitudinally extending groove 329 (Figure 6) is formed in the inner surface of the annular actuator 37 and is configured to receive the outer end of the worm gear lever arm 325 upon assembly of the handle 25 such that rotation of the annular actuator drives rotation of the worm gear 307 relative to the worm gear housing 301 (and hence the handle 25).

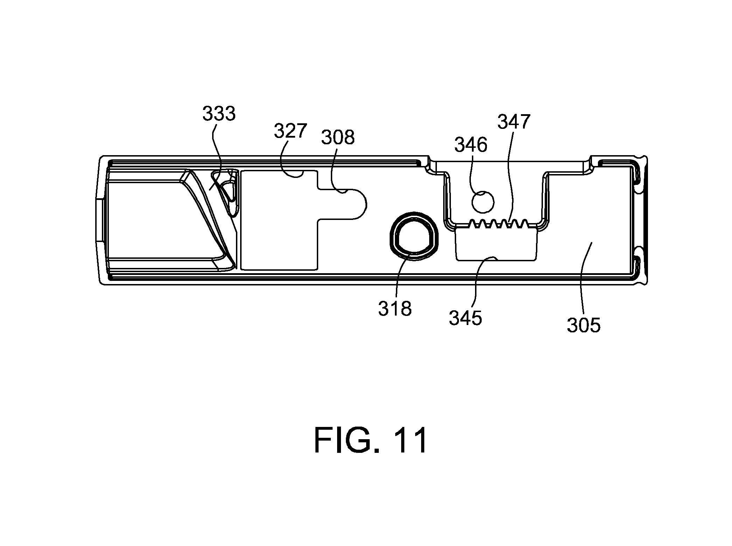

Suitable worm gear threads 331 project outward from the worm gear 301 (e.g., two sets of worm gear threads are illustrated in the various embodiments, such as in Figure 12) and seat within corresponding guide channels 333 (one of which is illustrated in Figure 11) formed in the respective inner surfaces of the top half 303 and bottom half 305 of the worm gear housing 301. The worm gear threads 331 and corresponding guide channels 333 are configured such that upon rotation of the worm gear 307 relative to the worm gear housing 301 (e.g., due to rotation of the annular actuator 37 from an initial or neutral position corresponding to the collapsed configuration of the electrode basket 33 to a rotated position corresponding to the expanded configuration of the electrode basket), the worm gear assembly 306 moves linearly (i.e., longitudinally) rearward relative to the worm gear housing.

As best seen in Figure 4, the shaft 313 of the linear bushing 309 is hollow. A threaded bore 335 extends transversely through the sidewall of the bushing head 311, with a substantially smaller bore 337 being formed longitudinally in the head of the bushing so as to provide communication between the hollow shaft 313 of the bushing 309 and the threaded bore formed in the head of the bushing. In this manner, the pull wire 39 associated with the electrode basket 33 extends into the worm gear housing 301 and through the worm gear 307 and bushing shaft 313. The pull wire 39 further extends through the smaller bore 337 in the head 311 of the bushing 309 into the threaded bore 335. A suitable fastener or rotatable plug 339 is disposed in the threaded bore 335 and captures (e.g., coils) the pull wire 39 to operatively connect the electrode basket 33 with the worm gear assembly 306, and hence the annular actuator 37 via the lever arm 325. The plug 339 also facilitates predetermined tensioning of the pull wire 39, e.g., upon assembly or subsequent adjustment of the handle. A notch 308 (Figure 10) is formed in the bottom half 305 of the worm gear housing 301 to provide access to the plug 339 even after assembly of the worm gear housing and barrel housing 201.

In operation, rotation of the annular actuator 37 from its initial position to its rotated position causes the worm gear assembly 306 to translate longitudinally rearward relative to the worm gear housing 301, thus further tensioning the pull wire 39 (i.e., broadly, acting on the control line) to effect expansion of the electrode basket 33 as illustrated in Figure 3. Rotation of the annular actuator 37 in the opposite direction releases the additional tension in the pull wire 39 to thereby allow the electrode basket 33 to return to its collapsed configuration (Figure 1).

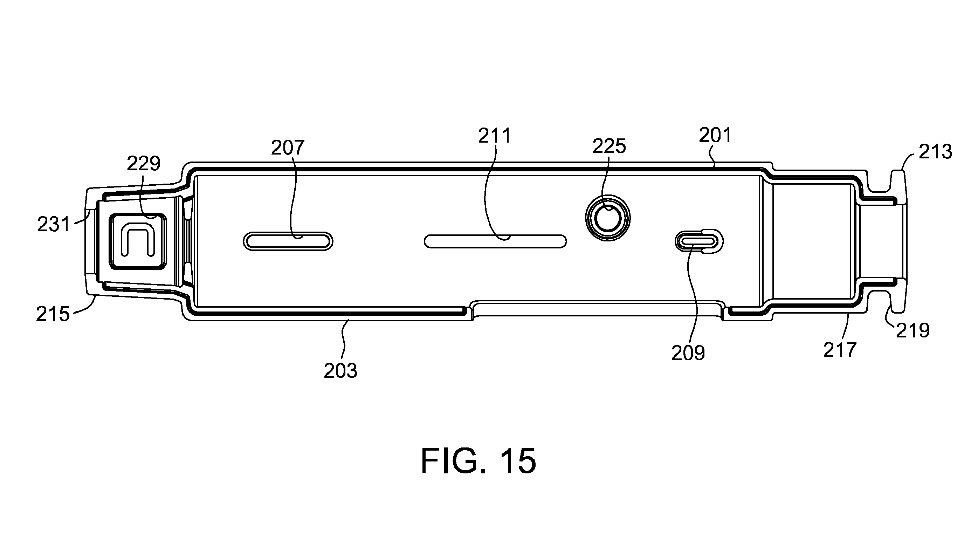

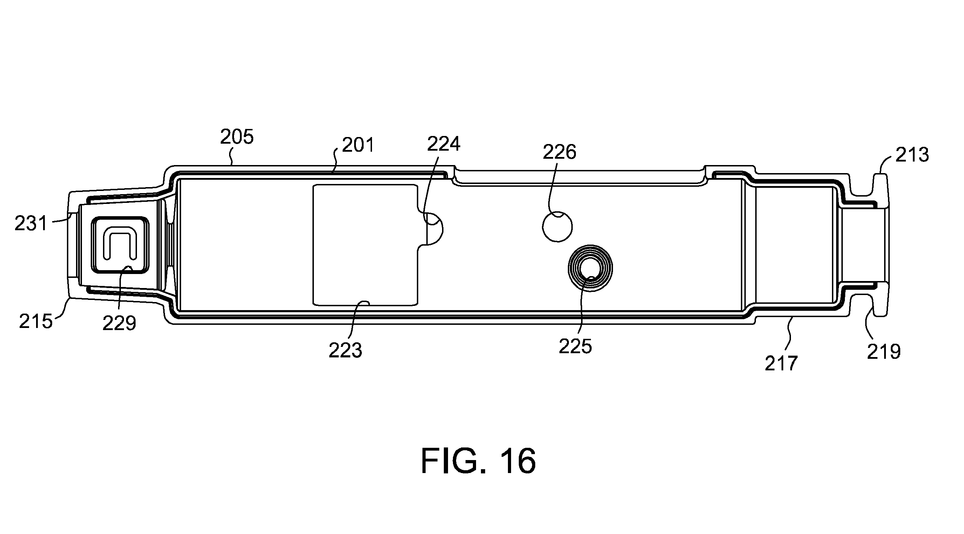

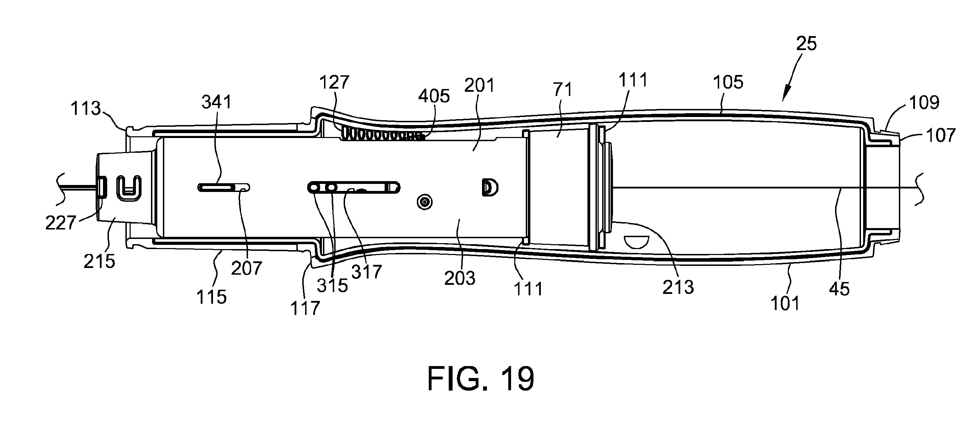

With general reference now to Figures 4, 6 and 15-21, the barrel housing 201 (broadly, the outer shuttle) is also of two-piece construction, including what is referred to herein as a top half 203 (Figure 15) and a bottom half 205 (Figure 16). In other suitable embodiments, the barrel housing 201 may be of single-piece construction, or constructed of more than two pieces. The top half 203 of the barrel housing 201, as best illustrated in Figure 15, includes a guide slot 207 for receiving a locating tab 341 (Figures 6, 9 and 9A) that projects radially outward from the outer surface of the top half 303 of the worm gear housing 301. This guide slot 207 is sized in length to accommodate sliding movement of the locating tab 341 - and hence the worm gear housing 301- relative to the barrel housing 201. A second locating tab 209 (Figure 15) extends radially inward from the inner surface of the top half 203 of the barrel housing 201 - in longitudinally spaced relationship with the guide slot 207 - and is receivable in a corresponding slot 343 (Figures 6 and 9) in the top half 303 of the worm gear housing 301. A third locating tab (not shown) extends radially inward from the inner surface of the top half 103 of the handle housing 101 and is receivable in a corresponding slot 211 (Figures 6 and 15) in the top half 203 of the barrel housing 201. Locating the various tabs 341, 209 (the third tab, projecting from the handle housing 101, not being shown) within the corresponding slots 207, 343, 211 in this manner also inhibits relative rotation of the respective housings 301, 201, 101 following assembly of the handle 25.

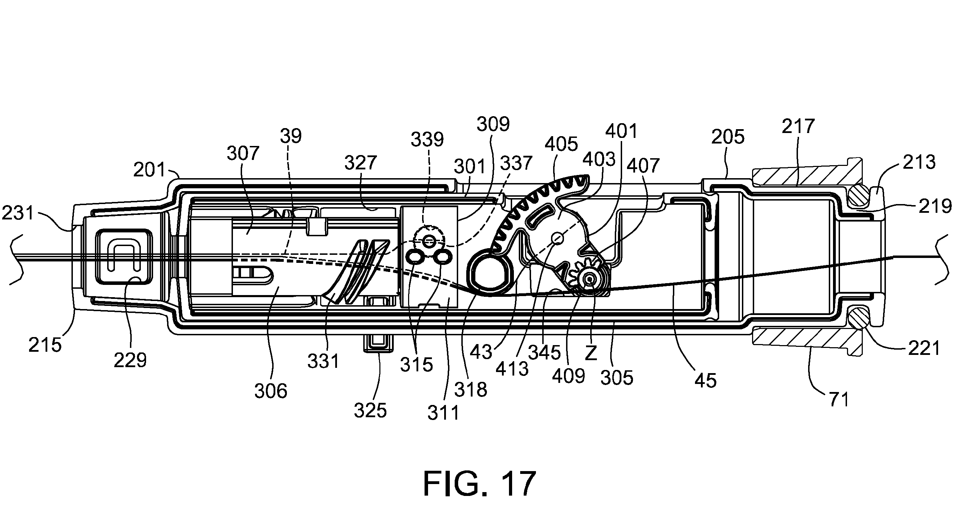

The illustrated barrel housing 201 has a longitudinally proximal or rear end 213, and a distal or front end 215. As seen best in Figures 17, 18 and 21A, a segment 217 of the barrel housing 201 adjacent its rear end 213 is sized in cross-section (e.g., in diameter in the illustrated embodiment) for slidable disposition within the slide bearing 71 to centrally position the barrel housing within the handle housing 101 while permitting sliding movement of the barrel housing (i.e., the outer shuttle) relative to the handle housing. An annular groove 219 is formed in the barrel housing segment 217 for seating an elastomeric sealing ring 221, such as an O-ring or other suitable sealing ring. The sealing ring 221 outer diameter is sized for sliding contact of the sealing ring with the inner surface of the slide bearing 71 upon sliding movement of the barrel housing 201 relative to the handle housing 101.

In a more particular embodiment, the elastomeric sealing ring 221 is generally loosely retained within the annular groove 219 of the barrel housing 201. Upon assembly of the barrel housing 201 and sealing ring 221 into the handle housing 101 (and hence in the slide bearing 71), the radially outer surface of the sealing ring is compressed by the slide bearing to generate friction between the sealing ring and the slide bearing to facilitate retention of the barrel housing at generally any position along the possible longitudinal travel of the barrel housing relative to the handle housing. In one particularly suitable embodiment, the friction between the sealing ring 221 and slide bearing 71 is sufficient to retain the barrel housing 201 at a desired longitudinal position, but sufficiently loose enough to permit the operator of the catheter system 21 to move the barrel housing using the slide actuator 41 (e.g., to deflect the catheter shaft 29) with one hand.

As one example, the elastomeric sealing ring 221 may be suitably constructed, and more suitably molded, of silicone. It is understood, though, that the sealing ring 221 may be constructed of another suitable elastomeric material or combination of materials without departing from the scope of this disclosure. The slide bearing 71 is suitably of single-piece molded plastic. For example, in one suitable embodiment the slide bearing 71 is constructed of an acetal material. In other embodiments, however, the slide bearing 71 may be made of other suitable materials or combination of materials.

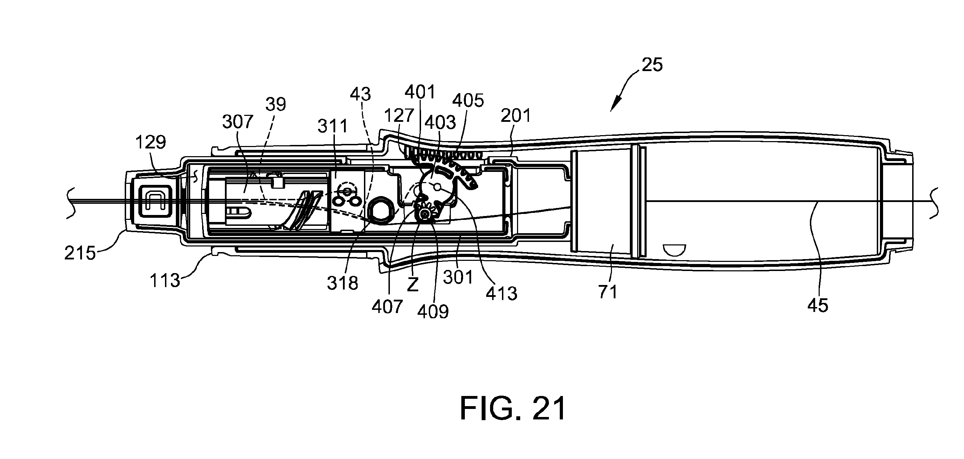

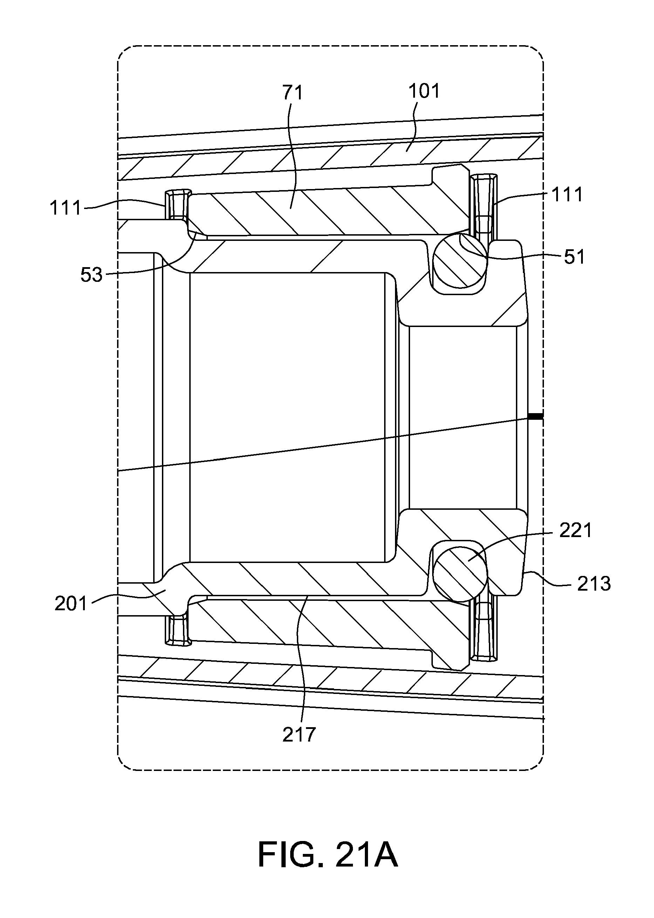

As illustrated in Figure 21A, the inner surface (e.g., inner diameter) of the slide bearing 71 according to one embodiment is chamfered 51 at its longitudinal back end and defines a neutral or initial position of the barrel housing 201 (corresponding to the undeflected configuration of the catheter). The chamfer 51 provides compression relief of the sealing ring 221 in the neutral position of the barrel housing 201 to inhibit compression set over long periods of non-use of the catheter system 21. The inner surface of the illustrated slide bearing 71 is also chamfered 53 at its longitudinal front end to provide a detent at the maximum longitudinal travel of the barrel housing 201 to thereby provide feedback to the operator that the barrel housing is at the maximum longitudinal travel thereof.

The bottom half 205 of the barrel housing 201 includes a window 223 (Figures 6 and 16) formed therein to accommodate passage of the worm gear lever arm 325 therethrough (see, e.g., Figure 17). The window 223 includes a notch 224 (Figure 16) that aligns with the notch 308 (Figure 10) of the bottom half 305 of the worm gear housing 301 to provide access to the plug 339 for adjusting the tension in the pull wire 39 even after assembly of the worm gear housing and barrel housing 201.

The top half 203 and bottom half 205 of the barrel housing 201 also have respective, opposed pin seats 225 (Figures 15 and 16) extending generally transversely inward from the respective inner surfaces thereof. These pin seats 225 are configured to pivotally retain the pinion member 401 (Figure 17) in the barrel housing 201 for longitudinal movement along with the barrel housing (i.e., the outer shuttle) relative to the handle housing 101.

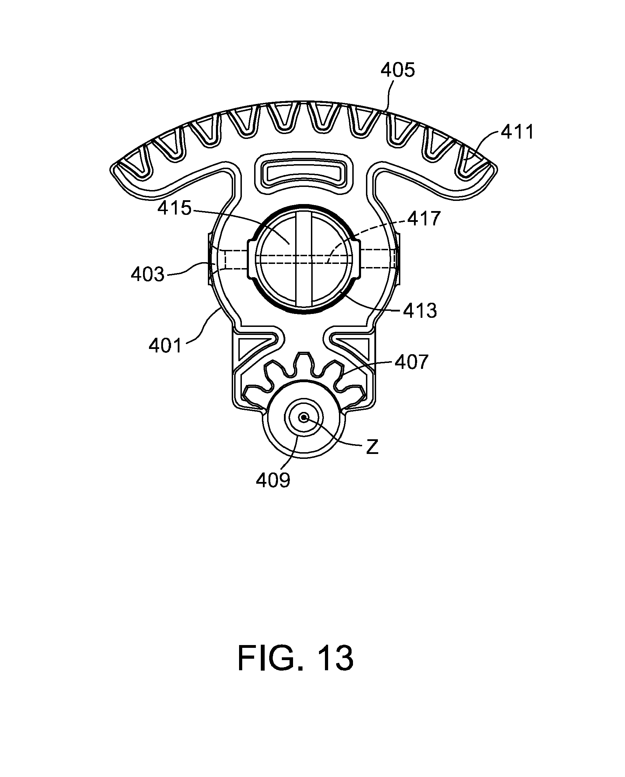

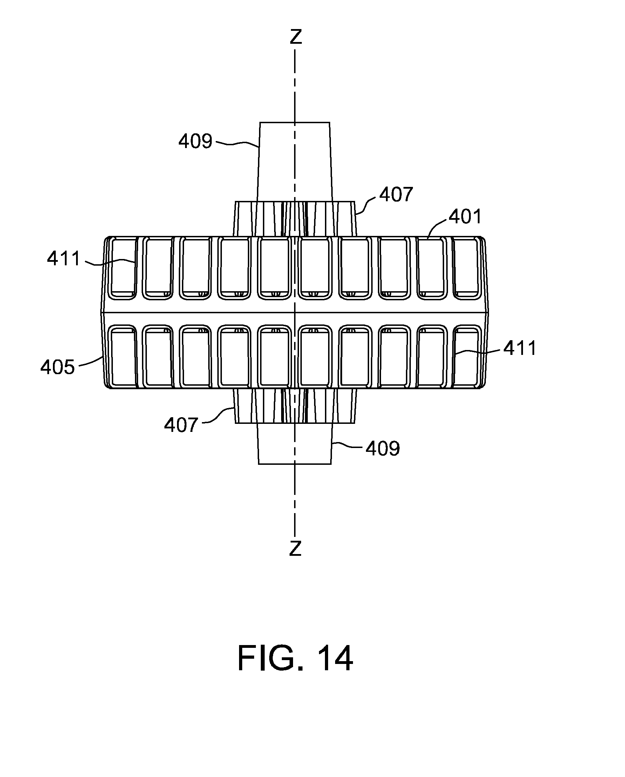

With particular reference to Figures 13 and 14, the pinion member 401 comprises a hub 403, a primary (broadly, a first) pinion gear 405 disposed on one transverse side of the hub, and a pair of secondary pinion gears 407 (broadly, a second or at least one second pinion gear) disposed on a transverse side of the hub opposite the primary pinion gear. A pin 409 extends outward from each of the respective secondary pinion gears 407 as illustrated in Figure 14 to define a pivot or rotation axis Z of the pinion member 401. Upon assembly of the handle 25, the pins 409 seat within the respective pin seats 225 of the barrel housing 201 to pivotally secure the pinion member 401 in the barrel housing 201 while permitting pivoting movement of the pinion member relative to the barrel housing about the pivot axis Z.

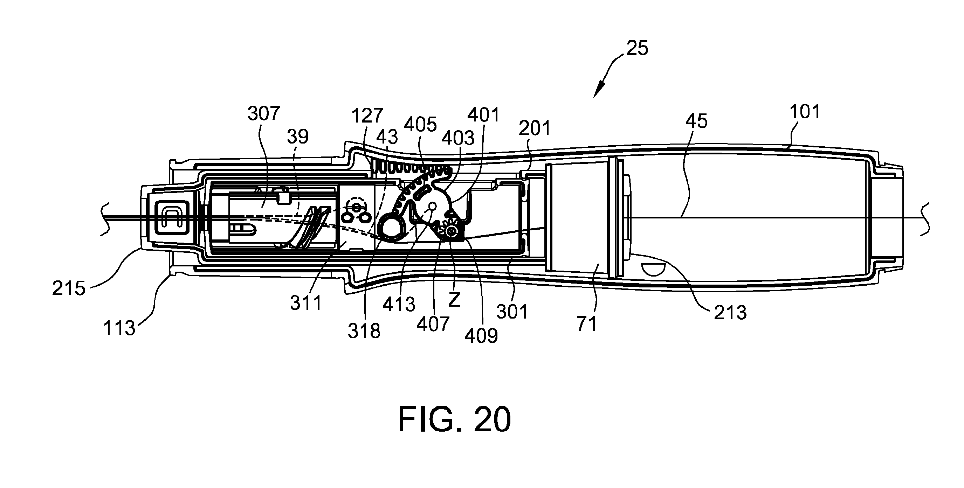

As seen best in Figure 14, the primary pinion gear 405 comprises two parallel rows of gear teeth 411. It is understood that in alternative embodiments the primary pinion gear 405 may comprise a single row of gear teeth extending the width of the primary pinion gear, or a single row of gear teeth extending less than the entire width of the primary pinion gear. In other alternative embodiments, the primary pinion gear 405 may comprise more than two rows of gear teeth. A respective corresponding rack 127 (one of which is illustrated in Figures 4 and 18-21, the other of which is not shown but is identical to that illustrated in these Figures) extends inward from the inner surface of each of the top half 103 and bottom half 105 of the handle housing 101. Accordingly, upon sliding movement of the barrel housing 201 (i.e., the outer shuttle) relative to the handle housing 101, the interengagement of the primary pinion gear teeth 411 with the corresponding racks 127 on the handle housing 101 cause the pinion member 401 to pivot relative to the barrel housing 201 about the pivot axis Z of the pinion member from an initial or neutral position (Figure 20) toward a maximum pivoted position (Figure 21).

A central bore 413 (Figure 13) extends at least into, and in the illustrated embodiment it extends through (e.g., from the top to the bottom of), the hub 403 of the pinion member 401. The bore 413 is threaded and receives a suitable fastener or rotatable plug 415 therein. A substantially smaller bore 417 extends transversely through the side of the pinion member hub 403 into open communication with the central bore 413. As illustrated in Figures 4 and 17, the pull wire 43 associated with deflection of the catheter shaft 29 extends into the handle housing 101, into the barrel housing 201 (and worm gear housing 301), and then through the smaller bore 417 in the hub 403 of the pinion member 401 into the central bore 413. The plug 415 is used to coil the pull wire 43 to thereby selectively tension the pull wire to a predetermined desired tension. Accordingly, the catheter shaft 29 is operatively connected to the barrel housing 201 (i.e., the outer shuttle) via the pinion member 401.

As illustrated in Figures 9 and 10, the pinion member 401 is disposed in part within the worm gear housing 301 upon assembly of the worm gear housing, with the pins 409 extending outward from windows 345 formed in the worm gear housing such that pins can seat in the respective pin seats 225 of the barrel housing 201. An opening 346 (Figures 10 and 10A) in the bottom half 305 of the worm gear housing 301 and corresponding opening 226 (Figure 16) in the bottom half 205 of the barrel housing 201 provide access to the plug 415 (see, e.g., Figure 10) to permit adjustment of the tension of the pull wire 43 even after assembly of the worm gear housing and barrel housing.

The central bore 413 of the pinion member 401 is offset from the pins 409 (and hence the pivot axis Z) such that upon pivoting movement of the pinion member about its pivot axis, the central bore 413 and plug 415 to which the pull wire 43 is connected orbits about the pivot axis of the pinion member. More particularly, in the initial or neutral position (Figures 17-20) of the pinion member 401 corresponding to the undeflected configuration of the catheter shaft 29, the primary pinion gear 405 and hence the central bore 413 and plug 415 of the pinion member are at a more longitudinally forward position relative to the barrel housing 201. In particular, as illustrated best in Figures 17 and 20, to facilitate proper alignment of the pinion member 401 in its neutral position a post 318 extends within the worm gear housing 301 between the opposed top half 303 and bottom half 305 of the worm gear housing. In the neutral position of the pinion member 401, the primary pinion gear 405 abuts against the post, with the rearwardmost teeth of the primary pinion gear intermeshed with the rearwardmost teeth of the rack 127 of the housing 101.

Upon sliding movement of the barrel housing (i.e., the outer shuttle) in a forward direction relative to the handle housing 101, the interengagement between the primary pinion gear teeth 411 and the racks 127 on the handle housing cause the pinion member to pivot about its pivot axis Z such that the primary pinion gear and hence the central bore 413 and plug 415 move in a generally rearward direction relative to the barrel housing 201 as illustrated in Figure 21. Because the catheter shaft 29 (to which the pull wire 43 is connected) is being moved longitudinally forward along with the barrel housing 201 but the plug 415 (to which the pull wire 43 is also connected) is pivoted rearward relative to the barrel housing, tension in the pull wire 43 increases and thus causes the deflectable segment of the catheter shaft 29 to deflect towards its maximum deflected configuration. Rearward movement of the barrel housing 201 causes the pinion member 401 to pivot back toward its neutral position, thus releasing the additional tension in the pull wire 43 to allow configuration of the catheter shaft 29 back toward its undeflected configuration.

The front end 215 of the barrel housing 201 includes a pair of detents 227 (Figure 8) extending radially outward from the housing. The slide actuator 41, as illustrated in Figures 6-8, has a central opening 55 sized for receiving the front end 215 of the barrel housing 201 therein. A pair of opposed guide channels 57 (Figure 8) extend longitudinally in the inner surface of the slide actuator 41 to accommodate the outward extending detents 227 of the barrel housing 201. A pair of shoulders 59 (Figure 8) are formed on the inner surface of the slide actuator 41 adjacent the longitudinally front end thereof.

To operatively connect the slide actuator 41 to the barrel housing 201 (i.e., the outer shuttle), the slide actuator is placed on the front end 215 of the barrel housing, with the detents 227 of the barrel housing disposed in and sliding along the channels 57 in the inner surface of the slide actuator. Upon further placement of the slide actuator 41 onto the barrel housing 201, the detents 227 become positioned just forward of the shoulders 59 formed in the inner surface of the slide actuator. The slide actuator 41 is then rotated relative to the barrel housing 201 so that the detents 227 seat on the shoulders 59 of the slide actuator to interlock and hence operatively connect the slide actuator to the barrel housing 201. In this manner, sliding movement of the slide actuator 41 relative to the handle housing 101 as illustrated in Figure 2 operatively slides the barrel housing (i.e., the outer shuttle) therewith, and hence pivots the pinion member 401 relative to the barrel housing to selectively configure the catheter between its undeflected and deflected configurations. It is understood that an outer shuttle may be configured other than as a housing such as the barrel housing 201 without departing from the scope of this disclosure, as long as the pull wire 43 (i.e., the control wire) associated with deflection of the catheter is operatively coupled with the outer shuttle such that movement of the outer shuttle relative to the handle acts on the catheter shaft 29, i.e., deflects the catheter shaft.

The catheter system 21 thus allows deflection of the catheter shaft 29 independent of selective configuration of the electrode basket 33 between its collapsed and expanded configurations using the annular actuator 37. However, because the worm gear housing 301 and worm gear assembly 306 (to which the electrode basket pull wire 39 is connected) are disposed within and carried by the barrel housing 201, the worm gear assembly and hence the connection point at which the electrode basket pull wire 39 is connected to the handle 25 move longitudinally forward relative to the handle housing 101 along with the barrel housing in response to actuation of the slide actuator 41. As a result, regardless of whether the electrode basket 33 is in its collapsed configuration or its expanded configuration, the length of the pull wire 39 from the electrode basket to the connection point on the worm gear assembly 306 is relatively shortened in response to actuation of the slide actuator 41 to deflect the catheter shaft 29. Accordingly, absent compensation for this shift, the pull wire 39 associated with the electrode basket 33 is susceptible to decreased tension and even the possibility of slack in the pull wire upon deflection of the catheter shaft 29. To this end, according to one embodiment herein the pull wire 39 associated with the electrode basket 33 is responsive to adjustment of the catheter shaft 29 configuration to thereby inhibit slack from forming in the electrode basket pull wire.

More particularly, in the illustrated embodiment the worm gear housing 301 - disposed within the barrel housing 201 and together with the worm gear assembly defining an inner shuttle of the handle 25 - includes a rack 347 (Figures 9, 9A, 10 and 10A) formed along respective edges of the windows 345 formed in top half 303 and bottom half 305 of the worm gear housing 301. As illustrated in Figures 9 and 10, the secondary pinion gears 407 interengage the respective racks 347 upon assembly of the worm gear housing 301 and barrel housing 201. The secondary pinion gears 407 and the corresponding racks 347 on the worm gear housing 301 are configured and arranged such that upon pivoting of the pinion member 401 relative to the barrel housing 201, the worm gear housing 301 and corresponding worm gear assembly 306 (i.e., the inner shuttle) are driven to move in a direction opposite the direction of movement of the barrel housing (i.e., longitudinally rearward in the illustrated embodiment). Stated another way, because the worm gear housing 301 and worm gear assembly 306 (i.e., the inner shuttle) are disposed within the barrel housing 201 and is thus moved longitudinally forward with the barrel housing relative to the handle housing 101 upon actuating the slide actuator 41 (and hence the barrel housing) forward, the secondary pinion gears 407 cause the worm gear housing 301 and assembly 306 to move longitudinally forward a distance less than the forward travel distance of the barrel housing. This is visible in Figure 21 by the gap 129 formed between the barrel housing 201 and the worm gear housing 301 when the barrel housing is moved to its maximum travel position corresponding to the deflected configuration of the catheter shaft 29.

By reducing the longitudinally forward travel of the worm gear housing 301 and assembly 306 (i.e., the inner shuttle) relative to the forward travel of the barrel housing 201 (i.e., the outer shuttle), slack is inhibited from forming in the electrode basket pull wire. As such, the worm gear housing 301 and assembly 306 (i.e., the inner shuttle) along with the secondary pinion gears 407 together broadly define a compensator assembly to which the electrode basket pull wire 39 (i.e., the control line) is operatively coupled and is responsive to actuation of the slide actuator 41 (and hence deflection of the catheter shaft 29) to inhibit slack from forming in the electrode basket pull wire. However, the worm gear housing 301 and assembly 306 must move forward some distance along with the barrel housing 201 to avoid increasing tension in the electrode basket pull wire 39 to a tension that would unintentionally expand the electrode basket. It is understood that in other embodiments a suitable compensator assembly other than a shuttle and pinion gear arrangement may be used to inhibit slack from forming in the electrode basket pull wire (i.e., the control line), as long as the compensator is responsive to actuation of the slide actuator 41 (i.e., deflection of the catheter shaft 29) to inhibit slack from forming in the electrode basket pull wire.

In one embodiment, the difference between the barrel housing 201 travel and the worm gear housing 301 travel is at least in part a function of the gear ratio between the primary pinion gear 405 and the secondary pinion gears 407, e.g., in view of the respective distances of the gears from the pivot axis Z of the pinion member 401. For example, in the illustrated embodiment the primary pinion gear 405 is suitably spaced from the pivot axis Z a distance greater than the distance of the secondary pinion gears 407 from the pivot axis. It is understood, however, that the gear ratio may be other than as described above without departing from the scope of this disclosure, as long as it is sufficient to inhibit slack from forming in the electrode basket pull wire 39.

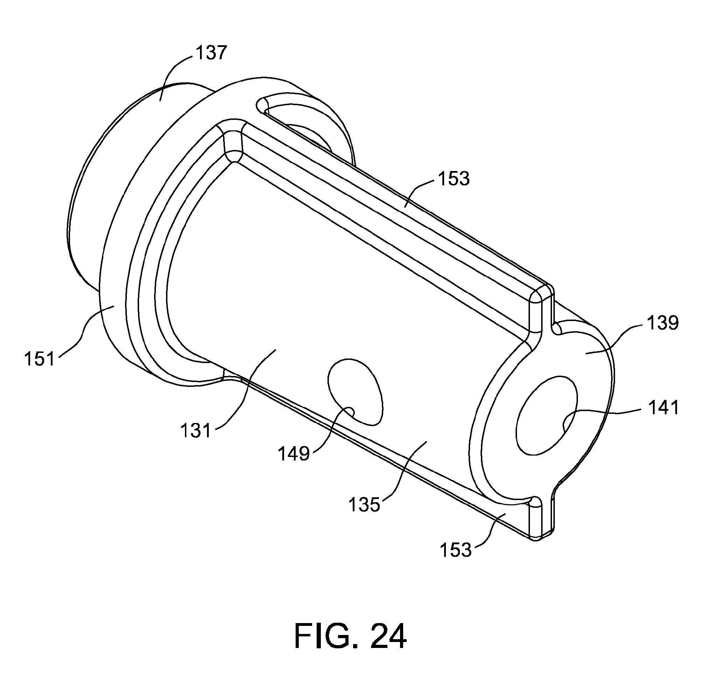

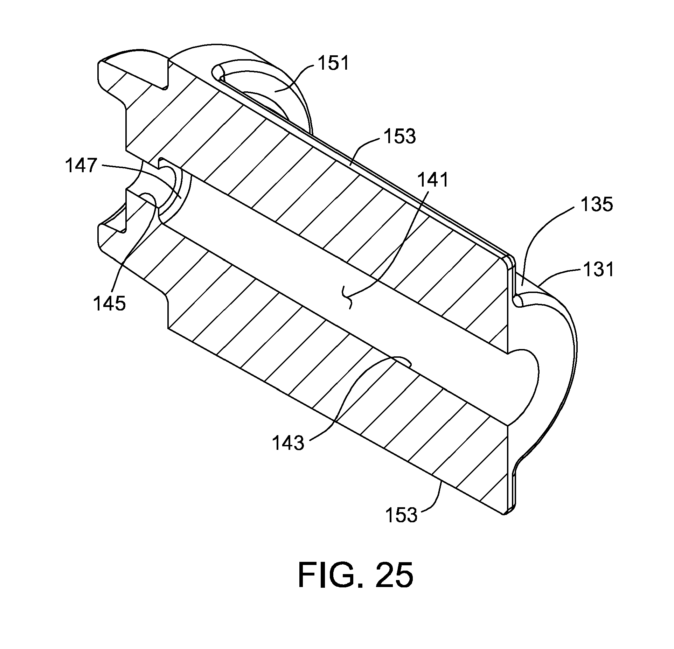

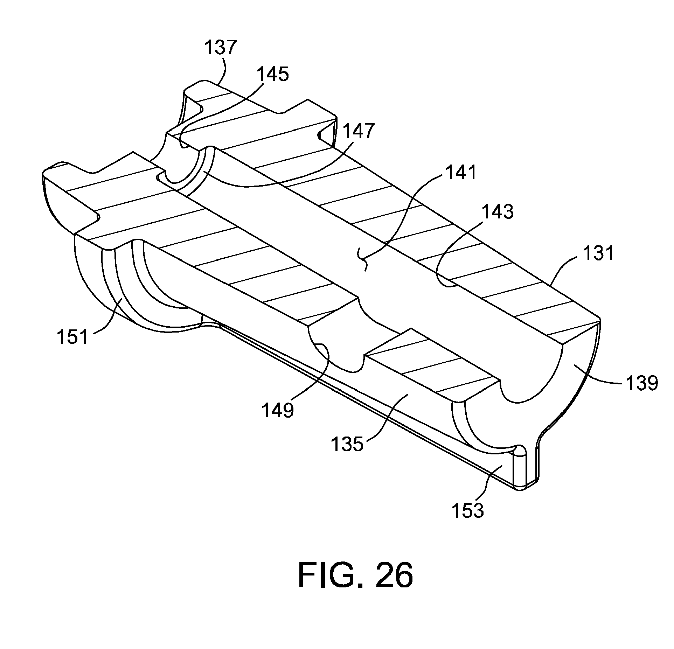

With reference now to Figures 7, 8 and 22-27, in one embodiment, the catheter 23 and more particularly the catheter shaft 29 is suitably connected to the handle 25 by a collar 131 (broadly a connector) and flex relief member 133. It is understood, however, that the flex relief member 133 may be omitted without departing from the scope of this disclosure. As illustrated in Figures 24-27, the collar 131 is generally tubular, having a frustoconical outer surface 135 tapering inward in cross-section from a longitudinally rear end 137 to a front end 139 thereof, and a central channel 141 extending the length of the collar and defining an inner surface of the collar. The central channel 141 includes a first segment 143 extending longitudinally from the front end 139 of the collar 131 and having a relatively greater transverse cross-section (i.e., diameter) to define a chamber for receiving the catheter shaft 29 into the collar, and a second segment 145 extending longitudinally from the rear end 137 of the collar and having a substantially smaller transverse cross-section than the first segment of the channel. A shoulder 147 is formed within the channel 141 by the reduced cross-section from the first to the second channel segments 143, 145 to thereby define a seat against which one end (i.e., the rear end) of the catheter shaft 29 abuts upon insertion of the catheter shaft into the collar 33 as illustrated best in Figures 7, 8 and 27.

In the illustrated embodiment, the first segment 143 of the channel 141 within collar 131 increases gradually (i.e., tapers outward) in transverse cross-sectional dimension (e.g., diameter) from the seat 147 to the front end 139 of the collar. More particularly, the diameter of the channel 141 at the seat 147 against which the shaft 29 abuts is substantially sized relative to the outer diameter of the catheter shaft to facilitate a close contact of the catheter shaft against the inner surface of the collar when the shaft abuts against the seat within the channel. Gradually increasing the transverse cross-sectional dimension of the channel 141 as it extends toward the front end 139 of the collar 131 provides a small clearance between the catheter shaft and the inner surface of the collar along a segment of the collar channel.

A port 149 is formed in and extends transversely through the sidewall of the collar 131 intermediate the front and rear ends 139, 137 of the collar, and more particularly at location along the channel segment where this is a small clearance between the inner surface of the collar and the outer surface of the catheter shaft. Upon assembly of the catheter shaft 29 with the collar 131, the catheter shaft is inserted longitudinally inward into the collar channel 141 at the front end 139 of the collar until the end of the catheter shaft abuts against the seat 147 formed within the channel. A suitable adhesive, such as a UV adhesive, is injected through the fill port 149 into the channel 141. The adhesive flows at least circumferentially around the outer surface of the catheter shaft 29 and in some embodiments also longitudinally within the segment of the channel 141 to fill the spacing between the catheter shaft outer surface and the relatively wider portion of the channel near the front end 139 of the collar (as well as some portions of the channel rearward of the fill port) to provide a circumferential bond between the collar and the catheter shaft. A uniform fill is controlled by the adhesive dispensing time. The collar 131 is suitably constructed of a material that permits the throughpassage of UV energy, such as a polycarbonate or other suitable material, to facilitate curing of the UV adhesive within the collar.

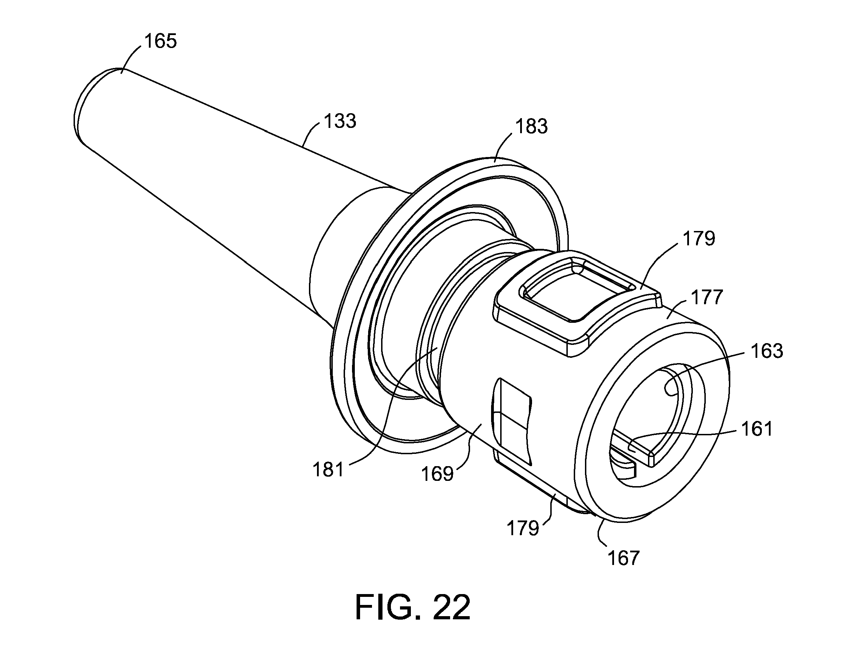

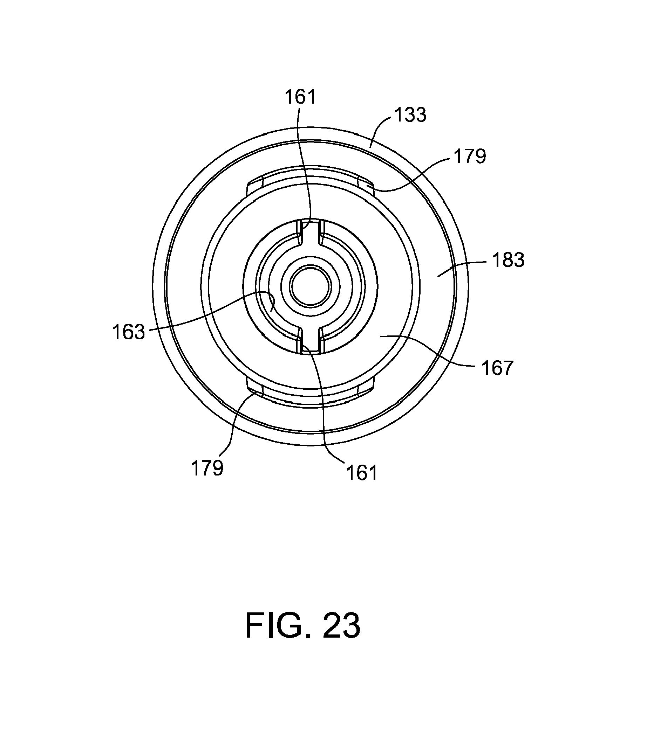

An annular flange 151 circumscribes the outer surface 135 of the collar 131 longitudinally inward from the rear end 137 of the collar, and more particularly at a location corresponding generally to the seat 147 formed within the collar channel 141. The flange 151 provides a stop for limiting longitudinal insertion of the collar 131 into the flex relief member 133 as best illustrated in Figures 7, 8 and 27. A pair of longitudinally extending flanges 153 (each broadly defining a projection) extend on the outer surface 135 of the collar 131 from the annular flange 151 to the front end 139 of the collar on opposite sides of the collar. These longitudinally extending flanges 153 are received in corresponding grooves 161 (Figures 22 and 23) of the flex relief member 133 as described in further detail below upon insertion of the collar 131 into the flex relief member to inhibit rotation of the collar (i.e., to provide torque resistance) relative to the flex relief member.

It is understood that a projection extending outward from the outer surface 135 of the collar 131 may be configured other than as a longitudinally extending flange 153, such as in the form of a post or other suitable projection. It is also contemplated that a single projection, or more than two projections, may be used within the scope of this disclosure. It is also understood that the catheter shaft 29 may be connected to the handle 25 in another suitable manner without departing from the scope of this disclosure.

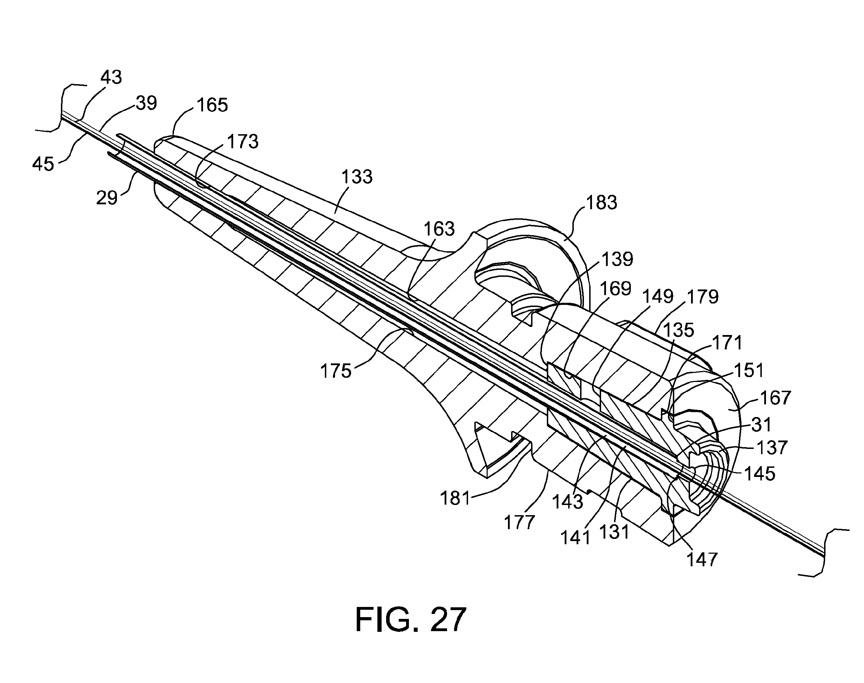

With reference to Figures 22, 23 and 27, the flex relief member 133 is generally tubular, having a central channel 163 extending the length of the flex relief member, e.g., from an open front end 165 to an open rear end 167 of the flex relief member. The central channel 163 of the illustrated flex relief member 133 comprises three particular segments. A collar housing segment 169 of the channel 163 extends longitudinally forward from the rear end 167 of the flex relief member 133, and is configured in accordance with the outer surface of the collar 131 so as to receive the collar into the collar housing segment in a generally close fitting relationship with the flex relief member (see, e.g., Figure 27). A seat 171 is formed generally at the rear end 167 of the flex relief member 133 to accommodate the annular flange 151 of the collar 131 to thereby facilitate proper longitudinal insertion of the collar into the flex relief member.

A grip segment 173 of the flex relief member channel 163 extends inward from the open front end 165 of the flex relief member 133 and is sized in transverse cross-section (e.g., diameter) for a close contact fit with the catheter shaft 29 within the flex relief member. In this manner, the catheter shaft 29 is inhibited against flexing at or near the collar 131 so as to inhibit the catheter shaft from being inadvertently disconnected or pulled out from the collar. An intermediate segment 175 of the flex relief member channel 163 extends longitudinally between the grip segment 173 and the collar housing segment 169 and is sized in transverse cross-section relatively larger than the cross-section of the catheter shaft 29. However, it is contemplated that the intermediate segment 175 could be sized for a closer fit of the catheter shaft 29 with the flex relief member 133 along this segment of the channel 163.

A mounting portion 177 of the flex relief member 133 is configured adjacent the rear end 167 thereof for being clamped by the barrel housing 201 of the handle 25 to retain the flex relief member on the handle. The illustrated mounting portion 177 includes a pair of generally square rib elements 179 disposed on opposite sides of the flex relief member 133. Corresponding pockets 229 (Figures 15 and 16) are disposed in the barrel housing 201 adjacent the front end 215 thereof for receiving the square rib elements 179 of the flex relief member 133. This facilitates alignment of the flex relief member 133 in the barrel housing 201 and inhibits rotation of the flex relief member relative to the barrel housing following assembly of the handle 25. An annular groove 181 is formed in the outer surface of the flex relief member 133 at the front end of the mounting portion 177 to receive a transversely inward extending flange 231 disposed at the front end 215 of the barrel housing 201 as illustrated best in Figures 7 and 8 to thereby positively retain the flex relief member on the barrel housing.

A generally frustoconical closure member 183 circumscribes the flex relief member 133 forward of the mounting portion 177 and is sized in transverse cross-section to seat within the front end of the slide actuator 41 to generally close the front end of the handle 25 upon assembly of the handle to thereby inhibit dirt or other debris from getting into the handle.

Although certain embodiments of this disclosure have been described above with a certain degree of particularity, those skilled in the art could make numerous alterations to the disclosed embodiments without departing from the spirit or scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the spirit of the disclosure as defined in the appended claims.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

As various changes could be made in the above constructions without departing from the scope of the disclosure, it is intended that all matter contained in the above description or shown in the accompanying drawings shall be interpreted as illustrative and not in a limiting sense.

## Claims

1. A catheter system comprising:
a handle,
an elongate hollow shaft having a proximal end connected to the handle and a distal end remote from the handle,
a first working component carried by the shaft and having at least one characteristic that is adjustable;
a first actuator associated with the handle for selectively adjusting at least one characteristic of the first working component;
a first control line extending at least in part within the shaft, the first control line operatively coupling the first actuator with the first working component such that actuation of the first actuator acts on the first control line to adjust the at least one characteristic of the first working component;
a second working component carried by the shaft and having at least one characteristic that is adjustable;
a second control line separate from the first control line and extending at least in part within the shaft, the second control line being operatively coupled to the second working component such that acting on the second control line adjusts at least one characteristic of the second working component; and
a compensator assembly associated with the handle, the second control line being operatively coupled to the compensator assembly, said compensator assembly being operable, in response to actuation of the first actuator, to inhibit slack from forming in the second control line.

2. The catheter system of claim 1 further comprising a second actuator associated with the handle and separate from the first actuator, the second control line operatively coupling the second actuator with the second working component such that actuation of the second actuator acts on the second control line to adjust the at least one characteristic of the second working component.

3. The catheter system of claim 1 or 2 wherein the handle has a longitudinal axis and comprises an outer housing, the compensator assembly comprising an inner shuttle disposed within the housing and slidable longitudinally relative thereto, the second control line extending from the second working component to the inner shuttle and being connected to the inner shuttle, the inner shuttle being at least in part responsive to actuation of the first actuator to slidingly move longitudinally relative to the housing to inhibit slack from forming in the second control line.

4. The catheter system of claim 1 or 2 wherein the handle has a longitudinal axis and comprises an outer housing, the first actuator at least in part comprising a pivoting member pivotable relative to the housing about an axis other than the longitudinal axis of the handle housing, the first control line extending from the first working component to the pivoting member and being connected to the pivoting member other than on the pivot axis thereof such that pivoting movement of the pivot member relative to the handle acts on the first control line to adjust the at least one characteristic of the first working component.

5. The catheter system of claim 1 or 2 wherein the handle has a longitudinal axis, the first actuator at least in part comprising a first shuttle moveable longitudinally of the handle, the first control line extending from the shaft to the first shuttle and being operatively connected to the first shuttle such that longitudinal movement of the first shuttle operatively acts on the first control line to adjust the at least one characteristic of the first working member.

6. The catheter system of claim 5 wherein the compensator assembly at least in part comprises a second shuttle moveable longitudinally of the handle relative to the first shuttle, the second control line extending from the second working component to the second shuttle and being operatively connected to the second shuttle, the second shuttle being responsive to longitudinal movement of the first shuttle to move longitudinally relative to the first shuttle to inhibit slack from forming in the second control line.

7. The catheter system of any one of claims 1 to 6 wherein the first working component comprises a segment of the shaft.

8. A method of controlling a catheter system of the type having a handle, a first working component operatively coupled to the handle by a first control line, and a second working component operatively coupled to the handle by a second control line separate from the first control line, the method comprising:
operating the handle to act on the first control line whereby acting on the first control line adjusts at least one characteristic of the first working component; and
acting on the second control line, in response to operating the handle to act on the first control line, to inhibit slack from forming in the second control line upon adjustment of the at least one characteristic of the first working component.

9. The method set forth in claim 8 further comprising operating the handle to act on the second control line whereby acting on the second control line adjusts at least one characteristic of the second working component other than to inhibit slack from forming in the second control line in response to adjustment of the at least one characteristic of the first working component.

10. The method set forth in claim 8 or 9 whereby operating the handle to act on the first control line comprises operating the handle to pull on the first control line whereby pulling on the first control line adjusts the at least one characteristic of the first working component.

11. The method set forth in claim 8 or 9 wherein the handle comprises a housing having a longitudinal axis, and a shuttle moveable longitudinally relative to the housing, the second control line extending from the second component to the shuttle and being connected to the shuttle, the step of acting on the second control line comprising automatically moving the shuttle longitudinally relative to the housing in response to acting on the first control line to adjust at least one characteristic of the first working component to thereby inhibit slack from forming in the second control line.

12. An electrode catheter system comprising:
a handle having a housing and a longitudinal axis,
an elongate, hollow flexible shaft having a proximal end connected to the handle and a distal end remote from the handle, a deflectable segment of the shaft being deflectable relative to a remaining segment of the shaft;
a shaft actuator associated with the handle for selectively deflecting the deflectable segment of the catheter shaft, the shaft actuator at least in part comprising a first shuttle disposable within and moveable longitudinally relative to the handle housing;
a shaft pull wire extending within the catheter shaft and being connected to the deflectable segment of the catheter shaft, the shaft pull wire being operatively coupled to the first shuttle such that actuation of the shaft actuator drives longitudinal movement of the first shuttle relative to the handle housing so as to pull on the shaft pull wire to deflect the deflectable segment of the catheter shaft;
an electrode component carried by the catheter shaft and being configurable from a collapsed configuration to an expanded configuration;
an electrode pull wire separate from the shaft pull wire and extending at least in part within the shaft and being operatively coupled to the electrode component;
a second shuttle disposed within and moveable longitudinally relative to the handle housing, the second shuttle also being moveable longitudinally relative to the first shuttle, the electrode pull wire being operatively coupled to the second shuttle, said second shuttle being responsive to longitudinal movement of the first shuttle to move longitudinally relative to said first shuttle so as to inhibit slack from forming in the electrode pull wire.

13. The electrode catheter system of claim 12 further comprising an electrode component actuator associated with the handle for selectively configuring the electrode component from its collapsed configuration to its expanded configuration, the electrode component actuator at least in part comprising an actuator assembly carried by the second shuttle, the electrode pull wire being connected to the actuator assembly carried by the second shuttle for conjoint longitudinal movement with the second shuttle.

14. The electrode catheter system of claim 12 or 13 further comprising a pivot member operatively coupling the first and second shuttles, the pivot member being responsive to longitudinal movement of the first shuttle to pivot relative to the first shuttle, pivoting movement of the pivot member driving longitudinal movement of the second shuttle relative to the first shuttle.

15. The electrode catheter system of claim 14 wherein the pivot member is carried by the first shuttle and has a pivot axis normal to the longitudinal axis of the handle housing, the pivot member having a primary pinion gear spaced from the pivot axis of the pivot member and a secondary pinion gear spaced from the pivot axis of the pivot member, the handle housing having a corresponding rack in interengagement with the primary pinion gear such that longitudinal movement of the first shuttle relative to the handle housing drives pivoting movement of the pivot member relative to the first shuttle, the second shuttle having a rack in interengagement with the secondary pinion gear such that pivoting movement of the pivot member drives longitudinal movement of the second shuttle relative to the first shuttle.
